(19) **Europäisches Patentamt / European Patent Office / Office européen des brevets**

(11) **EP 3 767 634 B1**

(12) # EUROPEAN PATENT SPECIFICATION

(45) Date of publication and mention
of the grant of the patent:
**12.06.2024 Bulletin 2024/24**

(51) International Patent Classification (IPC):
**G16H 30/40** *(2018.01)*     **G16H 50/50** *(2018.01)*
**G16H 50/70** *(2018.01)*

(21) Application number: **19187187.0**

(22) Date of filing: **19.07.2019**

(52) Cooperative Patent Classification (CPC):
**G16H 30/40; G16H 50/50; G16H 50/70**

(54) **METHOD AND SYSTEM FOR DATA AUGMENTATION USING SIMULATED CONTRASTS**

VERFAHREN UND SYSTEM ZUR DATENERWEITERUNG UNTER VERWENDUNG SIMULIERTER KONTRASTE

MÉTHODE ET SYSTÈME D'AUGMENTATION DES DONNÉES À L'AIDE DE CONTRASTES SIMULÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**20.01.2021 Bulletin 2021/03**

(73) Proprietor: **Siemens Healthineers AG**
**91301 Forchheim (DE)**

(72) Inventors:
• **KOBER, Tobias**
 **1007 Lausanne (CH)**
• **RICHIARDI, Jonas**
 **1202 Genève (CH)**

(74) Representative: **Fischer, Michael**
 **Siemens AG**
 **Postfach 22 16 34**
 **80506 München (DE)**

(56) References cited:
• **YU BITING ET AL: "Ea-GANs: Edge-Aware Generative Adversarial Networks for Cross-Modality MR Image Synthesis", IEEE TRANSACTIONS ON MEDICAL IMAGING, IEEE SERVICE CENTER, PISCATAWAY, NJ, US, vol. 38, no. 7, 1 July 2019 (2019-07-01), pages 1750-1762, XP011732657, ISSN: 0278-0062, DOI: 10.1109/TMI.2019.2895894 [retrieved on 2019-06-27]**

• **ORBES-ARTEAGA MAURICIO ET AL: "PADDIT: Probabilistic Augmentation of Data using Diffeomorphic Image Transformation", PROGRESS IN BIOMEDICAL OPTICS AND IMAGING, SPIE - INTERNATIONAL SOCIETY FOR OPTICAL ENGINEERING, BELLINGHAM, WA, US, vol. 10949, 15 March 2019 (2019-03-15), pages 109490S-109490S, XP060120435, ISSN: 1605-7422, DOI: 10.1117/12.2512520 ISBN: 978-1-5106-0027-0**

• **ZIZHAO ZHANG ET AL: "Translating and Segmenting Multimodal Medical Volumes with Cycle- and Shape-Consistency Generative Adversarial Network", ARXIV.ORG, CORNELL UNIVERSITY LIBRARY, 201 OLIN LIBRARY CORNELL UNIVERSITY ITHACA, NY 14853, 27 February 2018 (2018-02-27), XP081127686,**

• **SALOME KAZEMINIA ET AL: "GANs for Medical Image Analysis", ARXIV.ORG, CORNELL UNIVERSITY LIBRARY, 201 OLIN LIBRARY CORNELL UNIVERSITY ITHACA, NY 14853, US , vol. Version 1 13 September 2018 (2018-09-13), pages 1-44, XP081497021, Retrieved from the Internet: URL:https://arxiv.org/pdf/1809.06222v1.pdf [retrieved on 2020-01-22]**

• **HOO-CHANG SHIN ET AL: "Medical Image Synthesis for Data Augmentation and Anonymization using Generative Adversarial Networks", ARXIV.ORG, CORNELL UNIVERSITY LIBRARY, 201 OLIN LIBRARY CORNELL UNIVERSITY ITHACA, NY 14853, 26 July 2018 (2018-07-26), XP081119470,**

EP 3 767 634 B1

• **IGLESIAS JUAN EUGENIO ET AL: "Is Synthesizing MRI Contrast Useful for Inter-modality Analysis?", 22 September 2013 (2013-09-22), INTERNATIONAL CONFERENCE ON FINANCIAL CRYPTOGRAPHY AND DATA SECURITY; [LECTURE NOTES IN COMPUTER SCIENCE; LECT.NOTES COMPUTER], SPRINGER, BERLIN, HEIDELBERG, PAGE(S) 631 - 638, XP047041940, ISBN: 978-3-642-17318-9 * the whole document * * in particular * * sections 1-3 ***

**Description**

The present disclosure is directed, in general, to imaging techniques for medical image analysis of biological objects, like tissues, and more specifically to image denoising, recognition, segmentation and classification techniques in the medical domain, for instance in the field of Magnetic Resonance Imaging (MRI). More specifically, the present invention is related to data augmentation within the field of machine learning algorithms used for medical image analysis.

Many recent techniques in image denoising, recognition, segmentation and classification are based on deep neural networks, i.e. neural networks with (typically) many hidden layers. Following the corresponding "deep learning" paradigm, these networks have to be trained on large amounts of data - resulting also in the need to learn a large number of parameters which come with these deep network architectures (often several hundred thousand to tens or hundreds of millions of parameters). Consequently, many different instances (examples) of data to learn from have to be available in a labelled form; that is, the input training data has to be provided with the expected output to be presented to the network in its training phase. This is commonly applied in computer vision, where large amounts of labelled image data are available. As a simple example, one has easy access to millions of labelled cat images, i.e. images showing cats with the additional information "cat"/"no cat" (label); on these kinds of input data, a neural network can - nowadays pretty straightforwardly - be trained to identify a cat in an previously unseen image (in other words classify images in "contains cat" and "does not contain cat" classes).

In the medical imaging domain, however, much smaller amounts of labelled data are typically available. A common problem class in this domain is for instance image segmentation, i.e. to label parts of the medical image as belonging to a certain anatomical structure in a given organ or to a particular pathology (tumor, lesion, etc.). A very classical example for this use case would be to segment a brain image in its grey and white matter parts. In order to do so using a neural network, large amounts of already segmented brain images have to be presented to the network during the training phase. However, since these kinds of training data (the labels, to be more exact) are typically generated manually, very few data are actually available for training.

A paradigm which is often used to overcome the lack of training data in the medical image domain is "data augmentation". This is, changing existing labelled data in a way that it can be used as new training information. A very simple way of augmenting medical imaging data would be e.g. to translate or rotate the image (and if applicable, the corresponding label with it). Another classical approach to data augmentation is to apply certain changes to the image intensities to produce "new" labelled data, for example adding noise with different characteristics/strengths, to blur the image and/or to occlude part of the image. Many more, and more sophisticated data augmentation techniques have been proposed for different problems and domains, although most commonly the augmentation approaches applied are not specific to medical images, being directly adopted from practices in 2D computer vision. More recently, deformation-based augmentation techniques specific to medical imaging have been tested with good performance (see for instance Orbes Arteaga et al., PADDIT: Probabilistic Augmentation of Data using Diffeomorphic Image Transformation, abstract at SPIE medical imaging 2019). Another recent trend is to use high-capacity data-driven generative models such as generative adversarial networks (GANs) to create synthetic brain images, either from random noise, from existing brain images (possibly acquired with different sequences), or both. An example of a use of a GAN for cross-modality MR image synthesis is provided in the paper of Biting Yu et al. ("Ea-GANs: Edge-Aware Generative Adversarial Networks for Cross-Modality MR Image Synthesis", IEEE TRANSACTIONS ON MEDICAL IMAGING, vol. 38, No. 7, July 2019) . Another example of a use of a GAN for medical image synthesis for data augmentation is provided in the paper of Hoo-Chang Shin et al. ("Medical Image Synthesis for Data Augmentation and Anonymization using Generative Adversarial Networks", arxiv.org, arXiv:1807.10225, 26 July 2018, XP081119470) .

It is an objective of the present invention to propose a method and a system for automatically generating training datasets for improving medical image analysis by means of a machine learning model, wherein the generated training datasets present a high degree of variability (high number of uncorrelated data) allowing for an efficient and complete training of the machine learning model while keeping the generated data as "real" as possible.

Said objective is achieved according to the present invention by a method and a system for automatically generating training datasets for training a machine learning model of a medical imaging system used for medical image analysis according to the object of the independent claims. Dependent claims present further advantages of the invention.

The present invention proposes a method for automatically generating training datasets for medical image analysis by means of a machine learning model comprising a machine learning algorithm, said method comprising:

- acquiring a quantitative map of a biological object. By quantitative map it has to be understood an image of the biological object comprising pixels or voxels, wherein each pixel or voxel value is a quantitative value (i.e. a numerical value) representing a measure of at least one tissue characteristic/property of the biological object. Said quantitative map is preferentially acquired by means of, or from, an imaging system using a physical measurement procedure, wherein intensity and/or contrast variations within the quantitative map (i.e. the variations of said quantitative values of the pixels or voxels within the quantitative map) represent quantitative variations of said at least one tissue characteristic/property of the biological object. By "characteristic/property" it has to be understood any measurable or quantifiable property or characteristic of a tissue of the biological object;

- storing in a memory each acquired quantitative map;
- generating a training dataset comprising simulated images (also called synthetic images) of the biological object and for each simulated image, a corresponding label information;
- storing the training dataset in the memory;

the method being characterized in that the image contrast of each simulated image is computed/synthetized from one or more of said acquired quantitative maps and for at least one, preferentially each, of the acquired quantitative maps used for computing/synthetizing one or several simulated images, a label information is determined and used for deriving said corresponding label information for said one or several simulated images, e.g. for each of said simulated images, wherein a processing unit uses a predefined physical model for automatically computing/synthetizing the one or several simulated images from

- the quantitative values of the pixels or voxels of the acquired quantitative map(s) used for computing/synthetizing said one or several simulated images, and
- from at least one tunable simulation parameter, wherein said quantitative values and the tunable simulation parameter(s) are used as input in said predefined physical model and said one or several simulated images are generated by automatically tuning the tunable simulation parameter(s) so that one or several values of the tunable simulation parameter(s) are automatically inputted by the processing unit in the predefined physical model for computing/synthetizing said one or several simulated images.

According to the present invention, the one or several values of each tunable simulation parameter might be values that are predefined and stored in the memory, or values that are calculated by the processing unit according to a predefined calculation process stored for instance within the processing unit. According to the present invention, the processing unit is notably configured for automatically creating or deriving the corresponding label information for each simulated image from the label information determined for at least one of the acquired quantitative maps used for synthetizing/computing the simulated image. Said label information determined, for instance by the processing unit, for at least one of the acquired quantitative maps used to compute/synthetize the simulated image might be stored in the memory for said used quantitative map. The label information is configured for identifying a part within the quantitative map that belongs to an anatomical structure within the object or to a particular pathology with respect to the object. In particular, the acquired quantitative map might be directly labelled with said label information, or a derived image derived from the acquired quantitative map and comprising said label information might be determined by the processing unit and used as label information with respect to the quantitative map. The derived image is an image computed from the quantitative map and which enables for instance an easier labelling compared to a determination of a label performed directly on the quantitative map, while the derived image and the quantitative map still represent a same space. A derived image can be one of the simulated images obtained from computing the predefined physical model when using a set of tunable simulation parameters, said derived image becoming then automatically or manually labelled and being then used by the processing unit for automatically labelling all other simulated images obtained using the predefined physical model with other sets of tunable simulation parameters. The labelling of the quantitative map is preferentially carried out automatically by the processing unit, but might also be carried out at least in part manually. According to the present invention, a quantitative map or the derived image is preferentially labelled by a mask (i.e. a mask is automatically created by the processing unit from the quantitative map or from the derived image and then used by the processing unit for distinguishing one or several parts of the pixels or voxels of the quantitative map/derived image, each of said parts belonging for instance to an anatomical structure within the object or to a particular pathology with respect to the object and being labelled accordingly) or by a whole image label (i.e. the whole quantitative map or derived image is labelled or classified by the processing unit as belonging to an anatomical structure or particular pathology, e.g. "Alzheimer", "MS", or "broken bone", "normal bone" or similar. In the case of MRI, tunable simulation parameters are for instance sequence parameters like inversion time TI, repetition time TR, echo time TE.

The present invention proposes also a system for automatically generating a training dataset for medical image analysis by means of a machine learning model comprising a machine learning algorithm, the system comprising:

- optionally, an imaging system, like an MRI system or an X-ray imaging system, configured for acquiring by means of a physical measurement procedure a quantitative map of a biological object, wherein each quantitative map pixel or voxel value is a quantitative value representing a measure of at least one tissue characteristic/property of the biological object;
- an interface configured for enabling a processing unit to acquire the previously acquired quantitative maps;
- a memory for storing each quantitative map acquired by the processing unit, and for each acquired quantitative map, a label information;

- said processing unit configured for automatically generating a training dataset comprising simulated images of the biological object and for each simulated image a corresponding label information. Preferentially, the processing unit is configured for storing the training dataset in the memory. According to a preferred embodiment, the processing unit is further configured for automatically labelling one or several, preferentially each, quantitative maps acquired via said interface, and for storing for each of the labelled quantitative maps said label information. In particular, if the imaging system is not part of the claimed system, then the processing unit of the claimed system might be configured for collecting, from an imaging system or a database, the quantitative map(s) of the biological object through said interface;

characterized in that the processing unit is configured for automatically computing/synthetizing the image contrast of each simulated image from one or more, preferentially each, of said acquired quantitative maps, wherein the processing unit is configured for using a predefined physical model for automatically computing/synthetizing the simulated images from

- the quantitative values of the pixels or voxels of the acquired quantitative map(s) used for computing/synthetizing the simulated image; and
- from at least one tunable simulation parameter,

wherein said quantitative values and the tunable simulation parameter(s) are used as input in said predefined physical model and several simulated images are generated by the processing unit by automatically tuning the tunable simulation parameter(s), the processing unit being further configured for automatically creating or deriving the corresponding label information for each simulated image from the label information of the acquired quantitative map. According to the present invention, by tuning the tunable simulation parameters in the predefined physical model, i.e. by automatically inputting in the predefined physical model one or several values for at least one of said tunable simulation parameters, the processing unit is able to automatically generate several simulated images from a same set of quantitative maps. Advantageously, it can therefore use the label information of few quantitative maps, e.g. one or two quantitative maps for deriving the corresponding label information of several simulated images.

The obtained simulated images according to the present invention are realistic images representing the biological object, wherein quantitative variations of the contrast within each simulated image is obtained from real images (i.e. said quantitative maps) through said predefined physical model.

According to the present invention, the claimed system might be further configured for using the generated training dataset for training a machine learning algorithm of the machine learning model, wherein the simulated images are typically used as inputs in the machine learning algorithm and the corresponding label information as desired output. Preferentially, the trained machine learning algorithm is then used by the system according to the invention for analyzing, preferentially automatically, real images acquired, e.g. by the claimed imaging system, from other real biological objects.

The foregoing has outlined rather broadly the features and technical advantages of the present disclosure so that those skilled in the art may better understand the detailed description that follows. In particular, the present invention enables a simple and fast generation of simulated data (i.e. said simulated images) that can be used for training a machine learning algorithm. It is a new way for data augmentation that presents numerous advantages compared to existing techniques, notably because it is based on a "true" physical model, i.e. it enables to create simulated images that resemble real data closely.

For a more complete understanding of the present disclosure, and the advantages thereof, reference is now made to the following descriptions taken in conjunction with the accompanying drawings, wherein like numbers designate like objects, and in which:

Figure 1 illustrates a flowchart of a method for automatically generating training datasets according to the invention;

Figure 2 illustrates a system for implementing the claimed method;

Figures 3 and 4 show different simulated images obtained according to the present invention.

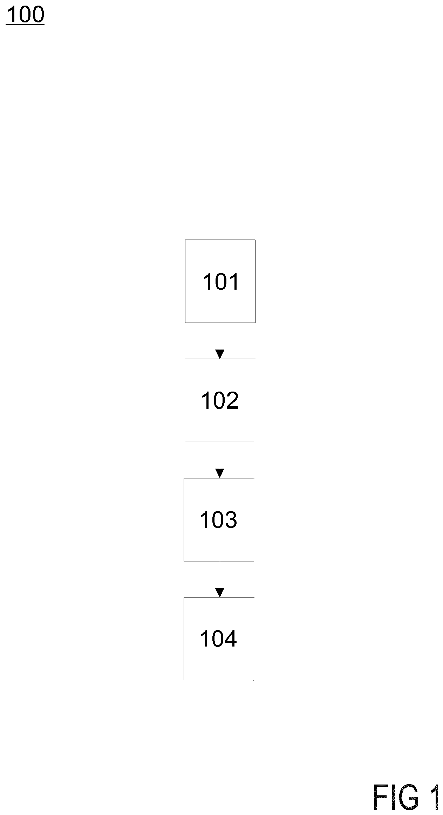

Figures 1 to 4, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged device. The numerous innovative teachings of the present application will be described with reference to exemplary non-limiting embodiments.

We will now describe in more details the method according to the invention through Figure 1 which describes the different steps of the method 100 carried out by the system according to the invention for automatically generating a training dataset.

At step 101, the system according to the invention acquires a real image of a biological object, like a brain. By real image it has to be understood a quantitative map of said biological object, wherein the quantitative map provides quantitative values regarding one or several tissue characteristics of the biological object. Said real image might be acquired by an imaging system part of the claimed system, or from an imaging system or database not part of the claimed system but to which the claimed system might be connected to through an interface for collecting/acquiring one or several real images. A typical example of imaging system is for instance an MRI system. According to the present invention, each quantitative map (or real image) according to the invention is acquired by carrying out a physical measurement procedure. According to a preferred embodiment, a processing unit might be connected to an imaging system via a dedicated interface in order to collect quantitative maps previously acquired by said imaging system.

While the present invention is particularly suited for MRI, it is not restricted to this specific field of technology and can apply to any imaging system and/or techniques wherein a predefined physical model might be used for synthetizing an image contrast from the acquired quantitative map(s) and one or several tunable simulation parameters.

In CT, for example, X-rays attenuated by the imaged object are sampled and reconstructed to tomographic images. In MRI, the spin resonance effect is used to sample the imaging data, here in the spatial frequency domain, and then reconstructed using corresponding methods. According to the present invention, the physical measurement procedure has to give rise to a real image wherein variations of the intensity and/or contrast represent variations of a biological (tissue) characteristic and are a function of the imaging parameters used for acquiring the real image by means of said physical measurement procedure. For instance, in X-ray imaging, variation of contrast and intensity is related to a physical attenuation factor (measured in Hounsfield units); in ultrasound imaging, the intensity also reflects a certain attenuation, but on a relative scale. In MRI, relaxation parameters in conjunction with the chosen acquisition technique and other residual factors determine the resulting image contrast.

At step 102, one or several quantitative maps of the biological object are stored by the processing unit in a memory of the system according to the invention. Preferentially, label information is stored for each quantitative map that has been acquired. Preferentially, and additionally, for each stored quantitative map, the system stores an information about the physical measurement procedure used for the quantitative map acquisition, as well as values of the imaging parameters that have been used for acquiring said quantitative map. Preferentially, said information and/or said values might be used in the predefined physical model. The label information might be automatically determined by the processing unit using known methods. Optionally, label information might be entered in the system according to the invention by a user for one or several quantitative maps.

At step 103, the processing unit of the system according to the invention generates a training dataset comprising several simulated images of the biological object, and for each simulated image a corresponding label information. According to the present invention, the system automatically inputs one or several quantitative maps $Q_i$, and one or several tunable simulation parameters $P_i$ into the predefined physical model which is configured for providing a known theoretical relation f between the quantitative maps $Q_i$, the tunable simulation parameter(s) $P_i$, and a simulated contrast S of the simulated image: $S = f(Q_i, P_i)$. An intensity in the obtained simulated image S represents therefore a conjunction of the predefined physical model f and the one or more quantitative maps $Q_i$ used to generate it. Typically, the predefined physical model is evaluated

**with** measured/quantitative values of one or several tissue characteristic(s) of the biological object (e.g. one or several values for the following biological object characteristic in case of MRI: longitudinal relaxation T1, transverse relaxation T2, and/or proton density PD) that correspond to pixel/voxel values of the quantitative map(s)
**for** a given set of tunable simulation parameters (e.g. one or several of the following sequence parameters in case of MRI: inversion time TI, repetition time TR, echo time TE). Finally, label information that has been stored together with one or several, preferentially each, acquired quantitative map is used for automatically generating said corresponding label information for each simulated image.

In MRI, for example, a first quantitative map Q1 might be a measurement of one of the two main relaxation parameters (T1 and T2) and a second quantitative map Q2 might be a measurement of the proton density (PD) for a biological object, like an organ. The quantitative maps Q1 and Q2 might be used to generate several simulated images $S = f(Q1, Q2, P_i)$ comprising artificial contrasts based on the relaxation parameter quantitative maps Q1, Q2 (i.e. spatial quantitative maps depicting the corresponding physical parameter at a given location in the organ) by tuning the tunable simulation parameter $P_i$, i.e. by automatically changing in the predefined physical model the values of the tunable simulation parameter $P_i$, so that each simulated image is obtained from the same set of quantitative maps, but with a different value for at least one of the tunable simulation parameters. For instance, based on a measurement of PD and T2 (i.e. quantitative maps acquired for PD and T2 holding the corresponding value at position *pos*), a simulated T2-weighed image with, as simulation parameter $P_i$, a virtual echo time TE, i.e. $P_i=TE$, can be generated through the following predefined physical model computing simulated image S as a function of Q1, Q2, and TE, i.e. S = f(Q1, Q2, TE) :

$$simulated\_intensity(pos) = PD(pos) * \exp\left(-\frac{TE}{T2(pos)}\right) \qquad \text{Eq. 1}$$

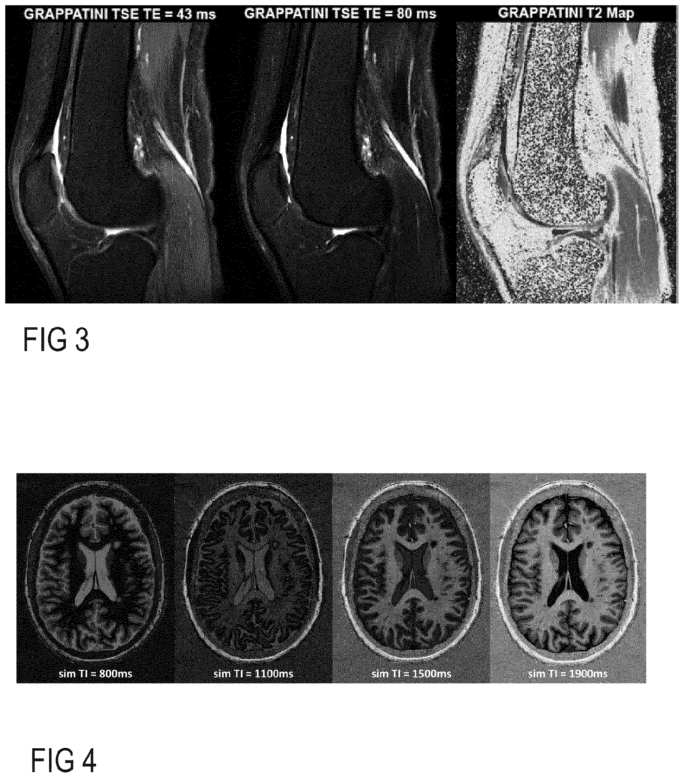

for every voxel at position pos, wherein PD(pos) is the value of PD at position pos within the quantitative map Q2 and T2(pos) is the value of T2 within the quantitative map Q1 at position pos. An example of two simulated contrasts is shown in Figure 3 (along with a corresponding T2 map). Similar formulas might be used for simulating T1-weighted images and others. An example of a series of simulated T1-contrasts based on a single PD and T1 map measurement is notably given in Figure 4. The obtained simulated images are then used for creating a training dataset for a machine learning algorithm.

At step 104, the training dataset comprising the simulated images and for each simulated image said corresponding label information is stored. Preferentially, said training dataset might be used by the system according to the invention for training a neural network to process medical images.

Advantageously, the simulated images obtained according to the present invention resemble real images much more than if contrasts and/or intensities of an existing image were just warped in a more or less random fashion. In the example provided through Eq. 1, many simulated T2-weighted images might be generated and fed to a neural network, making it therefore possible to create many training data based on only two quantitative maps (PD and T2).

Additionally, the present invention comprises automatically or manually labelling the quantitative map. In particular, labelling according to the present invention might include segmenting and/or marking the acquired quantitative map. Known techniques for labelling the quantitative maps might be used. According to the present invention, the label information might be associated to the whole quantitative map (i.e. all pixels or voxels of the quantitative map are associated to the same label - usually called whole image label) or for a part of the quantitative map (i.e. the label information distinguishes some pixels or voxels of the quantitative map from other pixels or voxels of the quantitative map). The same applies to the derived image. In order to obtain the derived image, the processing unit is configured for automatically deriving an image from the acquired quantitative map and for automatically labelling the derived image so that the derived image comprises said label information and is used by the processing unit as label information for the corresponding quantitative map. The resulting label information is preferentially used by the processing unit according to the invention to automatically identify one or several pixels or voxels within the quantitative map that indicate for instance a specific tissue characteristic/property/structure or a pathology. In particular, the processing unit is configured for automatically creating a mask comprising only said one or several identified pixels or voxels that enable to distinguish one or several parts of the quantitative map (or derived image) from other parts of said quantitative map (or derived image). A single mask can comprise a plurality of labels, i.e. different label information, if different sets of pixels or voxels have been identified as belonging to different anatomical structures of the biological object or to different pathologies. The mask according to the invention is a quantitative map mask comprising therefore the label information which has been determined for the quantitative map. Said mask is preferably used by the processing unit for automatically labelling and/or segmenting and/or marking all generated simulated images that are computed from the quantitative map from which the mask has been created. Typically, the processing unit is configured for applying the mask to the simulated image in order to identify within the simulated image pixels or voxels matching with the mask. This enables the processing unit to distinguish pixels or voxels of the simulated image that match or not with the mask and to automatically label accordingly the identified pixels or voxels. A first advantage of the present invention is for instance that a label information has to be generated only for the acquired quantitative maps used in the predefined physical model, and preferentially for instance for only one of the quantitative maps used in the predefined physical model, and not for the simulated images, since the processing unit is configured for using the label information of the quantitative map for automatically labelling the simulated images, using for instance the previously created quantitative map mask. For instance, in the example of Fig. 3, lesions could be manually marked (using a label) on PD and T2 quantitative maps for automatically creating a lesion mask by means of the processing system. Such a lesion mask is then used for all simulated contrast images obtained through Eq. 1, enabling to significantly increase the amount of training data. A second advantage compared to existing data augmentation techniques is that the predefined physical model used according to the present invention is preferentially non-linear. Consequently, the simulated image is not generated by a linear combination of existing images, or affine transforms as commonly used in image data augmentation. Consequently, the obtained simulated images according to the present invention increase the amount of new information at disposal for training the machine learning model, e.g. a neural network, which is advantageous for the training phase.

The imaging technique used for obtaining the quantitative map is no limiting. It could be quantitative maps obtained by means of MRI or based on another imaging technique. For instance, the present invention might be used for generating a training dataset based on a Computed Tomography (CT) quantitative map given in Hounsfield units, or based on a quantitative ultrasound map (e.g. stiffness map or similar). Optionally, the simulated images could even be generated from previously obtained artificial/simulated quantitative maps whose pixel or voxel values represent simulated values for one or several tissue characteristics of the biological object instead of using the real images obtained for the real biological object. The method according to the invention may comprise for instance generating an artificial quantitative map of a biological object like a virtual brain, and then generating, several simulated brain images according to the previously described method steps.

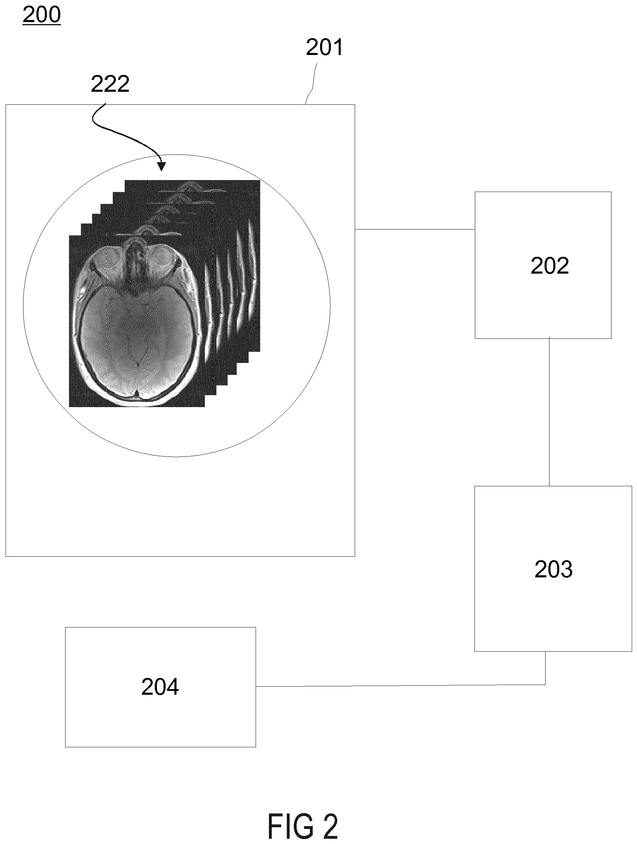

Figure 2 illustrates a system 200 for automatically generating training datasets for medical image analysis, for instance by means of a machine learning algorithm. The system 200 comprises:

- optionally, an imaging system 201, for acquiring one or several quantitative maps 222 of a biological object, wherein contrast/intensity variations within the maps represent variations of one or several tissue characteristics at some spatial locations within the biological object;
- an interface 202 for enabling a processing unit 204 to collect at least one, or each acquired quantitative map, wherein the interface 202 might also be used by the processing unit 204 for acquiring label information for each of the collected quantitative maps;
- a memory 203 for storing each collected quantitative map, and optionally, its corresponding label information if acquired through the interface 202;
- the processing unit 204, typically a processor or controller, configured for generating a training dataset comprising simulated images of the biological object and for each simulated image, a corresponding label information, the processing unit 204 being further configured for storing the training dataset in the memory 203. Optionally, the label information might be determined automatically by the processing unit 204 for one or several quantitative maps, preferentially for at least one or only one of the quantitative maps used in the predefined physical model, and automatically stored by processing unit 204 in the memory 203 for and in association with each quantitative map for which the label information has been determined.

The system 200 according to the invention is characterized in that it uses the acquired quantitative maps as input in a predefined physical model that is configured for modeling a physical and real relation between the quantitative map(s), the tunable simulation parameter(s), and an intensity of the simulated image.

Advantageously, the present invention makes it possible to easily and cheaply generate large amounts of training data using at starting point measured data coming from said quantitative maps. The claimed method creates then simulated images that are used in a training dataset as it was real acquisitions of quantitative maps. The label information automatically generated and associated to each of said simulated images enables an identification within the simulated image of a specific structure/property/pathology that is used for training the machine learning model. Indeed, each simulated image together with its associated label information is used to populate the training dataset. Such generation of a training dataset is far much cheaper than prior techniques requiring an acquisition of additional quantitative maps as training data. In contrast to traditional data augmentation techniques, the simulated/synthetic images have the advantage that they are based on a true physical model, i.e. the simulations (i.e. computing said predefined physical model) will result in images which resemble real data closely.

Another important difference of the proposed idea compared to existing data augmentation techniques which are based on a change of the contrast/noise characteristics of an acquired image is that, depending on the predefined physical model used to synthesize the simulated images, the generation can be highly non-linear. Since neural networks usually easily learn linear relations, the present invention proposes to create training dataset of synthetic images that do not exhibit a linear relationship, and which have therefore and advantageously a higher "information content": in other words, compared to existing techniques, a neural network will learn more information from the training dataset obtained according to the present invention than from another dataset artificially augmented.

## Claims

1. A method (100) for automatically generating a training dataset for medical image analysis by means of a machine learning model, the method comprising:

   - acquiring (101) at least one quantitative map of a biological object;
   - storing (102) in a memory each acquired quantitative map;
   - generating (103) a training dataset comprising simulated images of the biological object and for each simulated image, a corresponding label information;
   - storing (104) the training dataset;

   the method being **characterized in that** the image contrast of each simulated image is computed/synthetized from one or more of said acquired quantitative maps and for at least one, preferentially each, of the acquired quantitative maps used for computing/synthetizing the simulated images, a label information is determined and used for deriving said corresponding label information, wherein a processing unit uses a predefined physical model for automatically computing/synthetizing the simulated images from

- quantitative values of pixels or voxels of said one or more acquired quantitative maps used for computing/synthetizing the simulated images, and
- from at least one tunable simulation parameter, wherein said quantitative values and the tunable simulation parameter(s) are used as input in said predefined physical model and the simulated images are generated by automatically tuning the tunable simulation parameter(s), wherein the predefined physical model is configured for modeling a physical and real relation f between the quantitative map(s) Qi, the tunable simulation parameter(s) Pi, and an intensity S of the simulated image according to $S=f(Q_i,P_i)$.

2. The method according to claim 1, wherein one or several values of the tunable simulation parameter(s) are automatically inputted by the processing unit in the predefined physical model for computing/synthetizing the simulated images.

3. The method according to claim 2, wherein, the one or several values of each tunable simulation parameter are either values predefined in the memory or values calculated by the processing unit according to a predefined calculation process.

4. The method according to one of the claims 1 to 3, comprising automatically labelling the acquired quantitative map or a derived image with the label information, wherein said derived image is an image derived from the quantitative map.

5. The method according to claim 4, wherein the quantitative map or the derived image is labelled by a mask or by a whole image label.

6. The method according to one of the claims 1 to 5, comprising using the generated training dataset for training the machine learning model, wherein the simulated images are used as input in the machine learning model and the label information as desired outputs of the machine learning model.

7. The method according to claim 6, comprising using the trained machine learning model for automatically analyzing real medical images acquired for another biological object by the imaging system.

8. The method according to one of the claims 1 to 7, wherein the predefined physical model is non-linear.

9. System (200) for automatically generating a training dataset for medical image analysis by means of a machine learning model, the system comprising:

- optionally, an imaging system (201) for acquiring a quantitative map of a biological object;
- an interface (202) for enabling a transmission of the acquired quantitative map to a processing unit;
- a memory (203) for storing each quantitative map received by the processing unit from the imaging system;
- the processing unit (204) configured for automatically generating a training dataset comprising simulated images of the biological object and, for each simulated image, a corresponding label information;

**characterized in that** the processing unit (204) is configured for computing/synthetizing the image contrast of each simulated image from one or more of said acquired quantitative maps, the processing unit being further configured for automatically creating or deriving the corresponding label information for each simulated image from a label information determined for at least one of the acquired quantitative maps, wherein the processing unit (204) is configured for using a predefined physical model for automatically computing/synthetizing the simulated images from

- the quantitative values of the pixels or voxels of said one or more acquired quantitative maps, and
- from at least one tunable simulation parameter, wherein said quantitative values and the tunable simulation parameter(s) are used as input in said predefined physical model and the simulated images are generated by automatically tuning the tunable simulation parameter(s),

wherein the predefined physical model is configured for modeling a physical and real relation f between the quantitative map(s) Qi, the tunable simulation parameter(s) Pi, and an intensity S of the simulated image according to S=f (Qi, Pi).

10. System (200) according to claim 9, wherein the processing unit is further configured for automatically inputting in the predefined physical model one or several values for at least one of said tunable simulation parameters in order to automatically generate several simulated images from a same set of quantitative maps.

11. System (200) according to claim 10, wherein the one or several values of each tunable simulation parameter are either values predefined in the memory or values calculated by the processing unit according to a predefined calculation process.

12. System (200) according to one of the claims 9 to 11, wherein the processing unit is configured for automatically labelling the quantitative map or a derived image by a mask or by a whole image label, wherein said derived image is an image derived from the quantitative map.

13. System (200) according to claim 12, wherein the processing unit is configured for automatically creating the mask from the quantitative map or from the derived image and applying said mask to each simulated image computed/synthetized from said quantitative map in order to automatically determine said corresponding label information.

**Patentansprüche**

1. Verfahren (100) zum automatischen Erzeugen eines Trainingsdatensatzes für die medizinische Bildanalyse mithilfe eines Modells für maschinelles Lernen, wobei das Verfahren Folgendes umfasst:

   - Aufnehmen (101) mindestens einer quantitativen Abbildung von einem biologischen Objekt,
   - Speichern (102) jeder aufgenommenen quantitativen Abbildung in einem Speicher,
   - Erzeugen (103) eines Trainingsdatensatzes, der simulierte Bilder von dem biologischen Objekt und für jedes simulierte Bild eine entsprechende Kennzeichnungsinformation umfasst,
   - Speichern (104) des Trainingsdatensatzes,

   wobei das Verfahren **dadurch gekennzeichnet ist, dass** der Bildkontrast jedes simulierten Bildes aus einer oder mehreren der aufgenommenen quantitativen Abbildungen berechnet/synthetisiert wird und für mindestens eine, vorzugsweise alle der zum Berechnen/Synthetisieren der simulierten Bilder aufgenommenen quantitativen Abbildungen eine Kennzeichnungsinformation bestimmt und zum Ableiten der entsprechenden Kennzeichnungsinformation benutzt wird, wobei eine Verarbeitungseinheit ein vordefiniertes physikalisches Modell zum automatischen Berechnen/Synthetisieren der simulierten Bilder aus Folgendem benutzt:

   - quantitativen Werten für Pixel oder Voxel der einen oder der mehreren aufgenommenen quantitativen Abbildungen, die zum Berechnen/Synthetisieren der simulierten Bilder benutzt werden, und
   - aus mindestens einem abstimmbaren Simulationsparameter, wobei die quantitativen Werte und die abstimmbaren Simulationsparameter als Eingabedaten für das vordefinierte physikalische Modell benutzt und die simulierten Bilder durch automatisches Abstimmen der abstimmbaren Simulationsparameter erzeugt werden, wobei das vordefinierte physikalische Modell für das Modellieren einer physikalischen und echten Beziehung f zwischen den quantitativen Abbildungen Qi, den abstimmbaren Simulationsparametern Pi und einer Intensität S des simulierten Bildes gemäß S = f(Qi, Pi) konfiguriert ist.

2. Verfahren nach Anspruch 1, wobei ein oder mehrere Werte für den/die abstimmbaren Simulationsparameter zum Berechnen/Synthetisieren der simulierten Bilder von der Verarbeitungseinheit automatisch in das vordefinierte physikalische Modell eingegeben werden.

3. Verfahren nach Anspruch 2, wobei es sich bei dem einen oder den mehreren Werten für jeden abstimmbaren Simulationsparameter entweder um im Speicher vordefinierte Werte oder um Werte handelt, die von der Verarbeitungseinheit gemäß einem vordefinierten Berechnungsprozess berechnet werden.

4. Verfahren nach einem der Ansprüche 1 bis 3, das das automatische Kennzeichnen der aufgenommenen quantitativen Abbildung oder eines abgeleiteten Bildes mit der Kennzeichnungsinformation umfasst, wobei es sich bei dem abgeleiteten Bild um ein von der quantitativen Abbildung abgeleitetes Bild handelt.

**5.** Verfahren nach Anspruch 4, wobei die quantitative Abbildung oder das abgeleitete Bild mit einer Maske oder einer Ganzbildkennzeichnung gekennzeichnet wird.

**6.** Verfahren nach einem der Ansprüche 1 bis 5, das das Benutzen des erzeugten Trainingsdatensatzes zum Trainieren des Modells für maschinelles Lernen umfasst, wobei die simulierten Bilder als Eingabedaten für das Modell für maschinelles Lernen und die Kennzeichnungsinformation als gewünschte Ausgabedaten des Modells für maschinelles Lernen benutzt werden.

**7.** Verfahren nach Anspruch 6, das das Benutzen des trainierten Modells für maschinelles Lernen zum automatischen Analysieren realer medizinischer Bilder umfasst, die von dem Abbildungssystem für ein anderes biologisches Objekt aufgenommen wurden.

**8.** Verfahren nach einem der Ansprüche 1 bis 7, wobei das vordefinierte physikalische Modell nichtlinear ist.

**9.** System (200) zum automatischen Erzeugen eines Trainingsdatensatzes für die medizinische Bildanalyse mithilfe eines Modells für maschinelles Lernen, wobei das System Folgendes umfasst:

- wahlweise ein Abbildungssystem (201) zum Aufnehmen einer quantitativen Abbildung von einem biologischen Objekt,
- eine Schnittstelle (202) zum Ermöglichen einer Übertragung der aufgenommenen quantitativen Abbildung zu einer Verarbeitungseinheit,
- einen Speicher (203) zum Speichern jeder quantitativen Abbildung, die von der Verarbeitungseinheit aus dem Abbildungssystem empfangen wurde,
- wobei die Verarbeitungseinheit (204) so konfiguriert ist, dass sie automatisch einen Trainingsdatensatz erzeugt, der simulierte Bilder von dem biologischen Objekt und für jedes simulierte Bild eine entsprechende Kennzeichnungsinformation umfasst,

**dadurch gekennzeichnet, dass**
die Verarbeitungseinheit (204) so konfiguriert ist, dass sie den Bildkontrast jedes simulierten Bildes aus einer oder mehreren der aufgenommenen quantitativen Abbildungen berechnet/synthetisiert, wobei die Verarbeitungseinheit ferner so konfiguriert ist, dass sie für jedes simulierte Bild aus einer für mindestens eine der aufgenommenen quantitativen Abbildungen bestimmten Kennzeichnungsinformation automatisch die entsprechende Kennzeichnungsinformation erstellt oder ableitet, wobei die Verarbeitungseinheit (204) so konfiguriert ist, dass sie ein vordefiniertes physikalisches Modell zum automatischen Berechnen/Synthetisieren der simulierten Bilder aus Folgendem benutzt:

- quantitativen Werten für die Pixel oder Voxel der einen oder der mehreren aufgenommenen quantitativen Abbildungen, und
- aus mindestens einem abstimmbaren Simulationsparameter,

wobei die quantitativen Werte und die abstimmbaren Simulationsparameter als Eingabedaten für das vordefinierte physikalische Modell benutzt und die simulierten Bilder durch automatisches Abstimmen der abstimmbaren Simulationsparameter erzeugt werden, wobei das vordefinierte physikalische Modell für das Modellieren einer physikalischen und echten Beziehung f zwischen den quantitativen Abbildungen Qi, den abstimmbaren Simulationsparametern Pi und einer Intensität S des simulierten Bildes gemäß S = f(Qi, Pi) konfiguriert ist.

**10.** System (200) nach Anspruch 9, wobei die Verarbeitungseinheit ferner so konfiguriert ist, dass sie automatisch einen oder mehrere Werte für mindestens einen der abstimmbaren Simulationsparameter in das vordefinierte physikalische Modell eingibt und so aus dem gleichen Satz quantitativer Abbildungen automatisch mehrere simulierte Bilder erzeugt.

**11.** System (200) nach Anspruch 10, wobei es sich bei dem einen oder den mehreren Werten für jeden abstimmbaren Simulationsparameter entweder um im Speicher vordefinierte Werte oder um Werte handelt, die von der Verarbeitungseinheit gemäß einem vordefinierten Berechnungsprozess berechnet werden.

**12.** System (200) nach einem der Ansprüche 9 bis 11, wobei die Verarbeitungseinheit so konfiguriert ist, dass sie die quantitative Abbildung oder ein abgeleitetes Bild automatisch mit einer Maske oder einer Ganzbildkennzeichnung kennzeichnet, wobei es sich bei dem abgeleiteten Bild um ein von der quantitativen Abbildung abgeleitetes Bild handelt.

**13.** System (200) nach Anspruch 12, wobei die Verarbeitungseinheit so konfiguriert ist, dass sie aus der quantitativen Abbildung oder dem abgeleiteten Bild automatisch die Maske erstellt und die Maske auf jedes aus der quantitativen Abbildung berechnete/synthetisierte simulierte Bild anwendet und so automatisch die entsprechende Kennzeichnungsinformation bestimmt.

## Revendications

**1.** Procédé (100) pour générer automatiquement un ensemble de données d'entraînement pour l'analyse d'images médicales au moyen d'un modèle d'apprentissage automatique, le procédé comprenant :

- l'acquisition (101) d'au moins une carte quantitative d'un objet biologique ;
- le stockage (102) dans une mémoire de chaque carte quantitative acquise ;
- la génération (103) d'un ensemble de données d'entraînement comprenant des images simulées de l'objet biologique et, pour chaque image simulée, une information d'étiquette correspondante ;
- le stockage (104) de l'ensemble de données d'entraînement ;

le procédé étant **caractérisée en ce que** le contraste de l'image de chaque image simulée est calculé/synthétisé depuis l'une ou plusieurs desdites cartes quantitatives acquises et, pour au moins une et de préférence chacune, des cartes quantitatives acquises utilisées pour calculer/synthétiser les images simulées, une information d'étiquette est déterminée et utilisée pour dériver ladite information d'étiquette correspondante, dans lequel une unité de traitement utilise un modèle physique prédéfini pour calculer/synthétiser automatiquement les images simulées à partir

- des valeurs quantitatives des pixels ou voxels des une ou plusieurs cartes quantitatives acquises utilisées pour calculer/synthétiser les images simulées, et
- à partir d'au moins un paramètre de simulation réglable, dans lequel les valeurs quantitatives et le(s) paramètre(s) de simulation réglable(s) sont utilisés comme entrées dans ledit modèle physique prédéfini et les images simulées sont générées en réglant automatiquement le(s) paramètre(s) de simulation réglable(s), dans lequel le modèle physique prédéfini est configuré pour modéliser une relation physique et réelle f entre la (les) carte(s) quantitative(s) Qi, le(s) paramètre(s) de simulation réglable(s) Pi et une intensité S de l'image simulée selon $S=f(Q_i, P_i)$.

**2.** Procédé selon la revendication 1, dans lequel une ou plusieurs valeurs du/des paramètre(s) de simulation réglable(s) sont automatiquement entrées par l'unité de traitement dans le modèle physique prédéfini pour calculer/synthétiser les images simulées.

**3.** Procédé selon la revendication 2, dans lequel une ou plusieurs valeurs du/des paramètre(s) de simulation réglable(s) sont soit des valeurs prédéfinies dans la mémoire, soit des valeurs calculées par l'unité de traitement selon un procédé de calcul prédéfini.

**4.** Procédé selon chacune des revendications 1 à 3, comprenant automatiquement l'étiquetage de la carte quantitative acquise ou d'une image dérivée avec l'information d'étiquette, dans lequel ladite image dérivée est une image dérivée à partir de la carte quantitative.

**5.** Procédé selon la revendication 4, dans lequel la carte quantitative ou l'image dérivée sont étiquetées par un masque ou par une étiquette d'image entière.

**6.** Procédé selon l'une quelconque des revendications 1 à 5, comprenant l'utilisation de l'ensemble de données d'entraînement généré pour entraîner le modèle d'apprentissage automatique, dans lequel les images simulées sont utilisées comme entrées dans le modèle d'apprentissage automatique et les informations d'étiquette comme sorties désirées du modèle d'apprentissage automatique.

**7.** Procédé selon la revendication 6, comprenant l'utilisation du modèle d'apprentissage automatique entraîné pour analyser automatiquement les images médicales réelles acquises pour un autre objet biologique par le système d'imagerie.

8. Procédé selon l'une quelconque des revendications 1 à 7, dans lequel le modèle physique prédéfini est non linéaire.

9. Système (200) pour la génération automatique d'un ensemble de données d'entraînement pour l'analyse d'images médicales au moyen d'un modèle d'apprentissage automatique, le système comprenant :

   - optionnellement, un système d'imagerie (201) pour l'acquisition d'une carte quantitative d'un objet biologique ;
   - une interface (202) pour permettre une transmission de la carte quantitative acquise à une unité de traitement ;
   - une mémoire (203) pour le stockage de chaque carte quantitative reçue par l'unité de traitement à partir du système d'imagerie ;
   - l'unité de traitement (204) configurée pour générer automatiquement un ensemble de données d'entraînement comprenant les images simulées de l'objet biologique et, pour chaque image simulée, une information d'étiquette correspondante ;

   **caractérisée en ce que** l'unité de traitement (204) est configurée pour calculer/synthétiser le contraste de l'image de chaque image simulée à partir d'une ou plusieurs des cartes quantitatives acquises, l'unité de traitement étant également configurée pour créer ou dériver automatiquement l'information d'étiquette correspondante pour chaque image simulée à partir d'une information d'étiquette déterminée pour au moins l'une des cartes quantitatives acquises, dans lequel l'unité de traitement (204) est configurée pour utiliser un modèle physique prédéfini pour calculer/synthétiser automatiquement les images simulées à partir

   - des valeurs quantitatives des pixels ou voxels des une ou plusieurs cartes quantitatives acquises utilisées pour calculer/synthétiser les images simulées, et
   - à partir d'au moins un paramètre de simulation réglable,

   dans lequel lesdites valeurs quantitatives et le(s) paramètre(s) de simulation réglable(s) sont utilisés comme entrées dans ledit modèle physique prédéfini et les images simulées sont générées en réglant automatiquement le(s) paramètre(s) de simulation réglable(s), dans lequel le modèle physique prédéfini est configuré pour modéliser une relation physique et réelle f entre la (les) carte(s) quantitative(s) Qi, le(s) paramètre(s) de simulation réglable(s) Pi et une intensité S de l'image simulée selon S=f (Qi, Pi).

10. Système (200) selon la revendication 9, dans lequel l'unité de traitement est également configurée pour entrer automatiquement dans le modèle physique prédéfini une ou plusieurs valeurs pour au moins l'un des paramètres de simulation réglables afin de générer automatiquement plusieurs images simulées à partir d'un même ensemble de cartes quantitatives.

11. Système (200) selon la revendication 10, dans lequel une ou plusieurs valeurs de chaque paramètre de simulation réglables sont soit des valeurs prédéfinies dans la mémoire soit des valeurs calculées par l'unité de traitement selon un procédé de calcul prédéfini.

12. Système (200) selon l'une quelconque des revendications 9 à 11, dans lequel l'unité de traitement est configurée pour étiqueter automatiquement la carte quantitative ou une image dérivée par un masque ou par une étiquette d'image complète, dans lequel ladite image dérivée est une image dérivée à partir de la carte quantitative.

13. Système (200) selon la revendication 12 dans lequel l'unité de traitement est configurée pour créer automatiquement le masque à partir de la carte quantitative ou à partir de l'image dérivée et appliquer le masque à chaque image simulée calculée/synthétisée à partir de la carte quantitative afin de déterminer automatiquement l'information d'étiquette correspondante.

100

101

102

103

104

FIG 1

<u>200</u>

201

222

202

203

204

FIG 2

FIG 3

FIG 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ORBES ARTEAGA et al.** PADDIT: Probabilistic Augmentation of Data using Diffeomorphic Image Transformation. *SPIE medical imaging,* 2019
- **BITING YU et al.** Ea-GANs: Edge-Aware Generative Adversarial Networks for Cross-Modality MR Image Synthesis. *IEEE TRANSACTIONS ON MEDICAL IMAGING,* July 2019, vol. 38 (7
- **HOO-CHANG SHIN et al.** Medical Image Synthesis for Data Augmentation and Anonymization using Generative Adversarial Networks. *arxiv.org, arXiv:1807.10225,* 26 July 2018